# FASCICULE DE BREVET EUROPEEN

(11) **EP 0 932 414 B1**
(45) Date de publication et mention de la délivrance du brevet: **18.09.2002**
(21) Numéro de dépôt: 97943933.8
(22) Date de dépôt: 03.10.1997
(51) Int. Cl.: A61K 38/38, A23L 1/305, A61P 43/00

(54) **UTILISATIONS DE COMPOSITIONS A BASE D'alpha-LACTALBUMINE**
VERWENDUNGEN VON alpha-LACTALBUMIN ZUSAMMENSETZUNGEN
USES OF COMPOSITIONS WITH alpha-LACTALBUMEN BASE

(30) Priorité: 03.10.1996 FR 9612064
(43) Date de publication de la demande: 04.08.1999
(73) Titulaire: Laboratoire Oenobiol S.A., 75016 Paris (FR)
(72) Inventeur: HARANG, Benoît, F-92310 Sèvres (FR)
(74) Mandataire: Vialle-Presles, Marie José
(86) Numéro de dépôt international: FR9701752
(87) Numéro de publication internationale: WO98014204

(56) Documents cités:
- WO-A-91/10441
- US-A- 4 485 040
- US-A- 4 639 465
- US-A- 4 650 789
- W. HEINE ET AL.: "THE SIGNIFICANCE OF TRYPTOPHAN IN HUMAN NUTRITION" AMINO ACIDS, vol. 9, no. 3, 1995, VIENNE, AT, pages 191-205, XP000676393 cité dans la demande

## Description

La présente invention est relative à de nouvelles utilisations de l'α-lactalbumine ainsi qu'à des compositions la contenant, comme complément nutritionnel et comme médicament, dans la régulation du sommeil et de l'horloge biologique contrôlant le cycle veille/sommeil.

Le rythme circadien qui contrôle l'activité des plantes et des animaux, détermine quand et comment dormir. D'une durée de 2 minutes à 25 minutes, ce cycle recouvre les 24 heures qui composent la journée. Des douzaines d'horloges internes se chargent de synchroniser les organismes vivants, aussi bien intérieurement que par rapport à l'environnement. Ces rythmes internes contrôlent et coordonnent la production hormonale, la faim, l'humeur, la température du corps et le niveau d'énergie. Une horloge interne contrôle aussi le cycle veille/sommeil.

La glande pinéale (épiphyse) et l'hypothalamus contrôlent conjointement les cycles veille/sommeil. Sous l'effet de l'obscurité, l'épiphyse libère de la mélatonine. Le taux de mélatonine atteint son maximum entre 1 et 5 heures du matin. Puis à l'aube, la lumière inhibe la sécrétion de la mélatonine. La lumière pénètre dans le cerveau en empruntant la voie qui va de la rétine à l'épiphyse en passant pas le noyau suprachiasmatique, dans l'hypothalamus. L'épiphyse ralentit alors la production de mélatonine. Cette faculté de la glande pinéale est donc indispensable au maintien de nos rythmes circadiens.

Pour synthétiser de la mélatonine, l'épiphyse a besoin de précurseurs. Le tryptophane (Trp), acide aminé essentiel, est le précurseur naturel de la mélatonine après une première étape de transformation en sérotonine. Il est donc nécessaire que le *pool* de tryptophane soit suffisant au moment où l'épiphyse accroît son activité, c'est-à-dire pendant le sommeil.

La mélatonine provient de la sérotonine, neuromédiateur produit par les neurones à partir de tryptophane que l'organisme est incapable de synthétiser (acide aminé essentiel) et qui doit être fourni par l'alimentation. Mais l'utilisation de cet acide aminé au niveau des neurones pose un délicat problème d'interactions biologiques.

Le Trp, après digestion de la protéine en contenant, est véhiculé par le sang jusqu'au cerveau. Ce passage de la barrière hémato-encéphalique est un transport actif et le Trp est en compétition avec 5 autres acides aminés : valine, leucine, tyrosine, isoleucine et phénylalanine. L'ingestion de protides privilégie les 5 acides aminés neutres, car ils existent en forte proportion dans les protéines alors que le Trp est l'acide aminé le plus rare des protéines.

Une fois amené au neurone, le tryptophane est hydroxylé en 5-hydroxy tryptophane, lui-même décarboxylé en 5-hydroxytryptamine (sérotonine). La sérotonine va ensuite subir, au niveau de l'épiphyse, une N-acétylation puis une 5-méthylation, qui conduisent à la formation de N-acétyl-5-méthoxytryptamine, la mélatonine.

L'hydroxyindole-O-méthyltransférase (HIOMT), enzyme catalysant l'étape finale de la synthèse de mélatonine, se trouve surtout dans la glande pinéale. Son activité est abaissée par la lumière et cet effet est empêché par sympathectomie.

La sérotonine peut être catabolisée par des amine-oxydases et excrétée dans les urines sous forme d'acide 5-hydroxyindol-3-acétique.

La mélatonine est fortement lipophile et diffuse de l'épiphyse dans la circulation sanguine, atteignant rapidement les autres fluides biologiques, tels que la salive et les liquides cérébro-spinal, séminal, ovarien, folliculaire et amniotique. Il est reconnu que la concentration plasmatique en mélatonine est un bon marqueur de l'activité physiologique de l'épiphyse. La mélatonine est métabolisée dans le foie en son dérivé hydroxylé, qui est principalement conjugué avec l'acide sulfurique : sulfate de 6-hydroxymélatonine (l'absorption orale de la mélatonine conduit à l'effet de premier passage hépatique). Environ 1% de la mélatonine est excrétée dans l'urine sous sa forme native ; la plupart est éliminée sous forme de son métabolite le sulfate de 6-hydroxymélatonine.

Une étude publiée en 1991 a montré que le fait d'administrer par voie intraveineuse du tryptophane accroît la sécrétion de mélatonine (G. HAJAK et al., Pharmacopsychotherapy, 1991, **24**, 17-20, *The influence of intravenous L-tryptophan on plasma melatonin and sleep in men*).

D'autre part, de nombreuses études ont porté sur l'importance d'une déplétion en Trp dans les perturbations de l'humeur et de la balance veille/sommeil, ainsi que, inversement, sur l'influence de la diète protéique et des acides aminés sur le fonctionnement cérébral.

Une revue récente a montré l'intérêt des protéines laitières et particulièrement de l'α-lactalbumine comme source nutritionnelle de tryptophane (W. HEINE et al., Amino Acids, 1995, 9, 191-205, *The significance of tryptophan in human nutrition*).

Enfin, la Demande Internationale WO 91/10441 décrit des compositions comprenant un polypeptide à teneur élevée en tryptophane et de l'arginine et/ou de l'ornithine, ces acides aminés étant présents sous forme libre ou sous forme peptidique (enchaînement de 2 à 8 acides aminés) ; en particulier, l'administration de compositions comprenant, en combinaison, de l'α-lactalbumine et de l'ornithine, à raison de 5 à 20 g par jour d'α-lactalbumine et de 200 à 850 mg d'ornithine, a pour effet une régulation de l'humeur et du sommeil.

La Demanderesse a maintenant trouvé que, de manière inattendue, l'ingestion par voie orale, d'une composition à base d'α-lactalbumine, sans apport de sucre et non associée à d'autres aminoacides, et malgré la compétition avec les aminoacides neutres, n'empêche pas l'absorption du tryptophane au niveau de la barrière hémato-encéphalique et joue le rôle de précurseur de mélatonine, avec comme corollaire, l'augmentation, de manière significative du taux de mélatonine sécrété pendant la nuit ; ceci permet l'utilisation d'une telle composition comme régulateur du sommeil et de l'horloge biologique. Ceci justifie en particulier l'intérêt d'une supplémentation nutritionnelle en protéines riches en tryptophane, quelques dizaines de minutes avant l'endormissement.

La présente invention a pour objet l'utilisation d'une composition essentiellement constituée d'α-lactalbumine, à une dose unitaire comprise entre 100 mg et 250 mg, pour la préparation d'un complément nutritionnel devant être utilisé par voie orale, dans la régulation du sommeil et/ou l'horloge biologique contrôlant le cycle veille/sommeil.

De manière inattendue, l'utilisation par voie orale d'une telle composition, ne comprenant comme protéine que de l'α-lactalbumine, permet d'obtenir un apport en tryptophane journalier d'environ 30-70 mg, apte effectivement à réguler le sommeil et l'horloge biologique contrôlant le cycle veille/sommeil.

La présente invention a également pour objet l'utilisation d'une composition essentiellement constituée d'α-lactalbumine, à une dose unitaire comprise entre 375 mg g et 1 g, pour la préparation d'un médicament destiné à être utilisé dans le traitement par voie orale des troubles du sommeil et/ou des troubles de l'horloge biologique contrôlant le cycle veille/sommeil chez les humains.

La présente invention a également pour objet un complément nutritionnel apte à réguler le sommeil et/ou l'horloge biologique contrôlant le cycle veille/sommeil, caractérisé en ce qu'il est essentiellement constitué d'α-lactalbumine à un dosage unitaire compris entre 100 mg et 250 mg et d'au moins un adjuvant technologique.

On entend par dosage unitaire, la quantité d'α-lactalbumine présente dans une unité de prise de complément nutritionnel.

Par exemple, un tel complément nutritionnel qui comprend 100 à 250 mg d'α-lactalbumine par unité de prise, est administré par voie orale, à une posologie journalière de 2 à 4 unités, pour obtenir la quantité journalière recherchée (400 mg à 1 g).

La présente invention a également pour objet un médicament destiné au traitement des troubles de l'horloge biologique contrôlant le cycle veille/sommeil et/ou des troubles du sommeil, caractérisé en ce qu'il est essentiellement constitué d'α-lactalbumine à un dosage unitaire compris entre 375 mg et 1 g et d'au moins un adjuvant technologique.

Un tel médicament qui comprend 375 mg à 1 g d'α-lactalbumine par unité de prise, est administré par voie orale, à une posologie journalière de 2 à 4 unités, pour obtenir la quantité journalière recherchée (1,5 g à 4 g).

Selon un mode de réalisation avantageux dudit complément nutritionnel ou dudit médicament, l'α-lactalbumine se présente sous forme lyophilisée.

Ledit adjuvant technologique est adapté à la formulation choisie : capsule, comprimé, sachet ou solution buvable et est notamment sélectionné parmi les lubrifiants, tel que le stéarate de magnésium, les produits pour la solubilisation instantanée en solution aqueuse, les agents gélifiants.

### EXEMPLE 1 : Composition selon l'invention.

L'α-lactalbumine utilisée est soit sous forme purifiée, soit sous la forme d'une fraction protéique de lait de vache, dans laquelle la teneur en α-lactalbumine est de l'ordre de 50 % ; des gélules préparées à partir d'une telle fraction protéique ont notamment la composition suivante :

| | par gélule | par jour | |
|---|---|---|---|
| | | 2 gélules | 4 gélules |
| Fraction protéique laitière sous forme de poudre lyophilisée | 225 mg | 450 mg | 900 mg |
| Gélatine | 112,8 mg | | |
| Lactose | 7,52 mg | | |
| Stéarate de magnésium | 6,7 mg | | |

De manière plus spécifique, cette fraction protéique présente la répartition suivante en amino acides :

| Composition moyenne en acides aminés (g/100 g de protéine) | | | |
|---|---|---|---|
| Acide aspartique | 14,8 | Isoleucine | 5,7 |
| Thréonine | 8,7 | Leucine | 10,4 |
| Sérine | 7,6 | Tyrosine | 4,3 |
| Acide glutamique | 17,3 | Phénylalanine | 4,8 |
| Proline | 5,6 | Lysine | 7,8 |
| Glycine | 3,1 | Histidine | 2,6 |
| Alanine | 4,0 | Arginine | 3,6 |
| Valine | 6,8 | Cystéine | 4,4 |
| Méthionine | 2,0 | Tryptophane | 3,0 |

### EXEMPLE 2 : Rôle de précurseur de la mélatonine d'une composition selon l'Exemple 1.

### * Protocole :

Cinq sujets sont nécessaires à l'évaluation de « l'activité promélatonine » d'une composition selon l'exemple 1.

Cette activité est déterminée en dosant le métabolite urinaire de la mélatonine dans la première miction matinale.

C'est une étude monocentrique randomisée, réalisée en ouvert, incluant cinq sujets. Les effets de l'absorption de 2 doses d'α-lactalbumine avec ou sans supplémentation en hydrate de carbone sont analysés.

La durée de l'étude est de 10 jours.

L'étude est réalisée selon le protocole illustré au Tableau I ci-après :

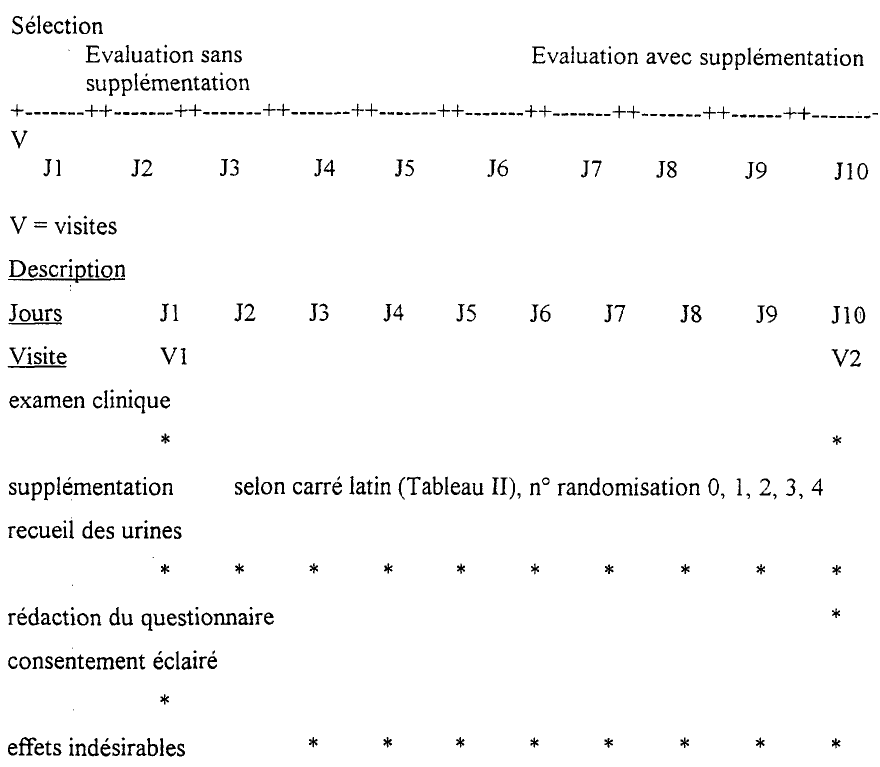

Chaque sujet reçoit une boîte de 10 sachets contenant 0 gélules pour 2 d'entre eux, 2 gélules pour 4 d'entre eux et 4 gélules pour les quatre autres afin d'assurer une prise journalière de : 0 gélule (0), 2 gélules (1 et 2), ou 4 gélules (3 et 4).

Les gélules sont absorbées avec un verre de lait tiède non sucré pour 1 et 3, ou sucré (l'équivalent d'un morceau n°4) pour 2 et 4, environ 1 heure avant l'heure prévue du coucher.

### * Résultats :

L'efficacité de ce traitement est déterminée par une augmentation de l'excrétion urinaire du sulfate de 6-hydroxymélatonine (6-SMT) après les différentes conditions de supplémentation (posologie, ingestion concomitante de sucre) décrites dans le Tableau II ou en l'absence de supplémentation, le sujet étant son propre témoin. Les traitements ont été répartis selon la méthode du carré latin.

**Tableau II :**

| Modèle de randomisation de l'étude | | | | | | | | | | |
|---|---|---|---|---|---|---|---|---|---|---|
| | J1 | J2 | J3 | J4 | J5 | J6 | J7 | J8 | J9 | J10 |
| S1 | 0 | 0 | 1 | 1 | 2 | 2 | 3 | 3 | 4 | 4 |
| S2 | 1 | 1 | 2 | 2 | 3 | 3 | 4 | 4 | 0 | 0 |
| S3 | 2 | 2 | 3 | 3 | 4 | 4 | 0 | 0 | 1 | 1 |
| S4 | 3 | 3 | 4 | 4 | 0 | 0 | 1 | 1 | 2 | 2 |
| S5 | 4 | 4 | 0 | 0 | 1 | 1 | 2 | 2 | 3 | 3 |
| S1 = sujet 1, S2 = sujet 2, S3 = sujet 3, S4 = sujet 4 ; 0 = pas de supplémentation, 1 = 2 capsules/jour sans sucre, 2 = 4 capsules/jour sans sucre, 3 = 2 capsules/jour avec sucre, 4 = 4 capsules/jour avec sucre. | | | | | | | | | | |

Les paramètres biologiques sont analysés à l'aide d'un test statistique de Student selon la méthode ANOVA (analyse factorielle).

Les quantités moyennes de 6-SMT excrétées dans l'urine et déterminées par RIA, par rapport à la concentration en créatinine urinaire, sont illustrées au Tableau III.

**Tableau III**

| dosage du 6-SMT (ng) par mg de créatinine (moyenne de deux dosages) et résultats de comparaison par ANOVA entre les quatre traitements | | | | | | | |
|---|---|---|---|---|---|---|---|
| Sujet | supplémentation en α-lactalbumine | | | | | ANOVA | |
| | 0 | 1 | 2 | 3 | 4 | F | p |
| S1 | 25,7 | 24,9 | 32,6 | 20,1 | 25,5 | 1,12 | 0,44 |
| S2 | 13,7 | 17,5 | 17,7 | 19,2¹ | 15,1 | 1,50 | 0,33 |
| S3 | 37,5 | 37,1 | 39,4² | 21,5 | 39,5 | 1,79 | 0,27 |
| S4 | 41,4 | 30 | 32,4³ | 22,3 | 37,5⁴ | 0,55 | 0,70 |
| S5 | 7,34 | 9,85 | 14,9⁵ | 10,5 | 11,5 | 2,80 | 0,14 |

| | | | | | | | |
|---|---|---|---|---|---|---|---|
| 1 : significatif *versus* 0 (90 %) ; | | | | | | | |
| 2 : significatif *versus* 3 (90 %), | | | | | | | |
| 3 : significatif *versus* 3 (90 %) ; | | | | | | | |
| 4 : significatif *versus* 3 (90 % ) ; | | | | | | | |
| 5 : significatif *versus* 0 (95 %), *versus* 1 (90 %). | | | | | | | |

Les mesures, qui sont analysées à l'aide d'un test statistique de Student selon la méthode ANOVA (analyse répétée) montrent que la sécrétion de mélatonine n'est pas influencée par le moment de supplémentation pour chaque sujet (F [facteur de probabilité (table de Student)] = 0,64, df [degré de liberté] = 4, p [probabilité] = 0,81).

L'analyse selon le test de Student selon la méthode ANOVA (analyse factorielle) révèle une augmentation significative de sécrétion en mélatonine pour 3 sujets (S1, S3 et S5) après 4 capsules/j et 1 sujet après 2 capsules/j.

Une prise d'hydrate de carbone n'a pas d'effet significatif sur l'excrétion urinaire de 6-SMT.

Pour l'un des sujets (S4), aucune différence significative n'a été observée après supplémentation et est due au problème qu'il est apparu que ce sujet absorbait un verre d'alcool avant de se coucher.

Les résultats obtenus suggèrent que l'administration orale d'α-lactalbumine (correspondant à 30-70 mg/j de tryptophane) augmente de manière significative l'excrétion urinaire de 6-SMT.

De manière surprenante, l'administration de tryptophane, sous la forme d'α-lactalbumine, par voie orale, à une concentration supérieure à 30-70 mg/j, augmente de manière significative la sécrétion en mélatonine chez les sujets sains.

## Revendications

1. Utilisation d'une composition essentiellement constituée d'α-lactalbumine, cette protéine n'étant pas associée à des acides aminés libres ni à des peptides riches en arginine ou en ornithine, à une dose unitaire comprise entre 100 mg et 250 mg, pour la préparation d'un complément nutritionnel devant être utilisé par voie orale dans la régulation du sommeil et/ou l'horloge biologique contrôlant le cycle veille/sommeil.

2. Utilisation d'une composition essentiellement constituée d'α-lactalbumine, cette protéine n'étant pas associée à des acides aminés libres ni à des peptides riches en arginine ou en ornithine, à une dose unitaire comprise entre 375 mg et 1 g, pour la préparation d'un médicament destiné à être utilisé dans le traitement par voie orale des troubles du sommeil et/ou des troubles d'horloge biologique contrôlant le cycle veille/sommeil chez les humains.

3. Complément nutritionnel apte à réguler le sommeil et/ou l'horloge biologique contrôlant le cycle veille/sommeil, **caractérisé en ce qu'**il est essentiellement constitué d'α-lactalbumine à un dosage unitaire compris entre 100 mg et 250 mg, ladite α-lactalbumine n'étant pas associée à des acides aminés libres ni à des peptides riches en arginine ou en ornithine, et d'au moins un adjuvant technologique.

4. Médicament destiné au traitement des troubles de l'horloge biologique contrôlant le cycle veille/sommeil et/ou des troubles du sommeil, **caractérisé en ce qu'**il est essentiellement constitué d'α-lactalbumine à un dosage unitaire compris entre 375 mg et 1 g, ladite α-lactalbumine n'étant pas associée à des acides aminés libres ni à des peptides riches en arginine ou en ornithine, et d'au moins un adjuvant technologique.

5. Complément nutritionnel selon la revendication 3 ou médicament selon la revendication 4, **caractérisés en ce que** l'α-lactalbumine se présente sous forme lyophilisée.

## Claims

1. Use of a compound essentially consisting of α-lactalbumin, this protein not being associated with free amino acids or with peptides rich in arginine or in omithine, at a unit dose of between 100 mg and 250 mg, for the preparation of a food supplement that must be taken orally in the regulation of sleep and/or of the biological clock that controls the waking and sleeping cycle.

2. Use of a compound essentially consisting of α-lactalbumin, this protein not being associated with free amino acids or with peptides rich in arginine or in ornithine, at a unit dose of between 375 mg and 1 g, for the preparation of a medicinal product to be used in the oral treatment of sleep disorders and/or disorders of the biological clock that controls the waking and sleeping cycle in humans.

3. Food supplement able to regulate sleep and/or the biological clock that controls the waking and sleeping cycle, **characterized in that** it consists essentially of α-lactalbumin at a unit dose of between 100 mg and 250 mg, the said α-lactalbumin not being associated with free amino acids or with peptides rich in arginine or in ornithine, and of at least one technological adjuvant.

4. Medicinal product intended for the treatment of disorders of the biological clock that controls the waking and sleeping cycle, **characterized in that** it consists essentially of α-lactalbumin at a unit dose of between 375 mg and 1 g, the said α-lactalbumin not being associated with free amino acids or with peptides rich in arginine or in ornithine, and of at least one technological adjuvant.

5. Food supplement according to claim 3 or medicinal product according to claim 4, **characterized in that** the α-lactalbumin is presented in a lyophilised form.

## Patentansprüche

1. Verwendung einer Zusammensetzung, die im Wesentlichen aus α-Lactalbumin besteht, wobei dieses Protein weder mit freien Aminosäuren noch mit Peptiden, die an Arginin oder Ornithin reich sind, assoziiert ist, in einer Dosiseinheit zwischen 100 mg und 250 mg zur Herstellung einer Nahrungsergänzung, die auf oralem Weg zur Regulierung des Schlafs und/oder der biologischen Uhr, die den Wach/Schlaf-Zyklus kontrolliert, zu verwenden ist.

2. Verwendung einer Zusammensetzung, die im Wesentlichen aus α-Lactalbumin besteht, wobei dieses Protein weder mit freien Aminosäuren noch mit Peptiden, die an Arginin oder Ornithin reich sind, assoziiert ist, in einer Dosiseinheit zwischen 375 mg und 1 g zur Herstellung eines Arzneimittels, dass dazu bestimmt ist, in der Behandlung von Störungen des Schlafs und/oder Störungen der biologischen Uhr, die den Wach/Schlaf-Zyklus bei den Menschen kontrolliert, auf oralem Weg verwendet zu werden.

3. Nahrungsergänzung, die zur Regulierung des Schlafs und/oder der biologischen Uhr, die den Wach/Schlaf-Zyklus kontrolliert, geeignet ist, **dadurch gekennzeichnet, dass** sie im Wesentlichen aus α-Lactalbumin in einer Dosiseinheit zwischen 100 mg und 200 mg, wobei das α-Lactalbumin weder mit freien Aminosäuren noch mit Peptiden, die an Arginin oder Ornithin reich sind, und mindestens einem technologischen Adjuvanz besteht.

4. Arzneimittel, dass zur Behandlung von Störungen der biologischen Uhr, die den Wach/Schlaf-Zyklus kontrolliert, und/oder Schlafstörungen bestimmt ist, **dadurch gekennzeichnet, dass** es im Wesentlichen aus α-Lactalbumin in einer Dosiseinheit zwischen 375 mg und 1 g, wobei das Lactalbumin weder mit freien Aminosäuren noch mit Peptiden, die an Arginin oder Ornithin reich sind, assoziiert ist, und wenigstens einem technologischen Adjuvanz besteht.

5. Nahrungsergänzung nach Anspruch 3 oder Arzneimittel nach Anspruch 4, **dadurch gekennzeichnet, dass** das α-Lactalbumin sich in lyophilisierter Form darstellt.
